(19) Europäisches Patentamt / European Patent Office / Office européen des brevets

(11) **EP 2 989 979 B1**

(12) **EUROPEAN PATENT SPECIFICATION**

(45) Date of publication and mention of the grant of the patent:
**26.01.2022 Bulletin 2022/04**

(21) Application number: **14182446.6**

(22) Date of filing: **27.08.2014**

(51) International Patent Classification (IPC):
**A61B 5/087** *(2006.01)*    **A61B 5/00** *(2006.01)*

(52) Cooperative Patent Classification (CPC):
**A61B 5/087; A61B 5/4818; A61B 5/7246; A61B 5/7275; G16H 50/30**

(54) **Respiratory waveform recognition method and system**

Verfahren und System zur Erkennung einer Atemwegswellenform

Procédé et système de reconnaissance de formes d'ondes respiratoires

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**02.03.2016 Bulletin 2016/09**

(73) Proprietor: **Apex Medical Corp.**
**New Taipei City, 23679 (TW)**

(72) Inventors:
 • **Lin, Kang-Ping**
  **23679 New Taipei City (TW)**
 • **Lin, Geng-Hong**
  **23679 New Taipei City (TW)**
 • **Jan, Hao-Yu**
  **23679 New Taipei City (TW)**
 • **Huang, Sheng-Cheng**
  **23679 New Taipei City (TW)**
 • **Shin, Po-Chung**
  **23679 New Taipei City (TW)**
 • **Yu, Ching-Liang**
  **23679 New Taipei City (TW)**
 • **Lee, Da-Long**
  **23679 New Taipei City (TW)**

(74) Representative: **Schwerbrock, Florian**
 **Hagenauer Strasse 1**
 **10435 Berlin (DE)**

(56) References cited:
 WO-A9-2013/138071    CN-A- 103 961 105
 US-A1- 2012 125 337

EP 2 989 979 B1

## Description

## FIELD OF THE INVENTION

The present disclosure relates to respiration state recognition and more particularly to a respiratory waveform recognition method and a system thereof, in which at least a portion of a respiratory airflow is fit to at least one of a curve, a weighted curve and a standard waveform.

## BACKGROUND OF THE INVENTION

Conventionally, when a patient fails to acquire sufficient oxygen concentration during respiration (hereinafter referred to as abnormal respiration state), he or she may use a respiration machine to maintain the necessary respiration activity to ensure sufficient oxygen concentration supply.

The abnormal respiration state usually occurs when the patient is sleeping and may generally be categorized into apnea, hypopnea or flow limitation, as described in detail below.

Apnea refers to decreased blood oxygen in patients due to the cessation of airflow supply to lung for more than 10 seconds during sleep. Patients diagnosed with sleep apnea suffer from abnormal life and increased risk of cardiovascular diseases, heart attack and cerebral haemorrhage due to poor sleep quality.

Hypopnea refers to reduced airflow due to overly slow or shallow breathing during sleep. In addition, it may be accompanied by oxygen desaturation and arousal for tens of seconds. For example, if during sleep the respiration activity and airflow are reduced by 50% to 70% and the blood oxygen saturation is reduced by 4%, hypopnea can be properly diagnosed.

Flow limitation refers to reduced airflow passage due to partially obstructed airway. Specifically, events of repeated partial or complete obstruction of the upper airway may occur during sleep and result in temporary arousal of the patient because of dysfunctional upper airway muscles, thickening of larynx soft tissues, tonsil proliferation or tonsil hypertrophy.

Apnea-hypopnea index (AHI), which is the total number of apneas and hypopneas per hour, may be used for determining hypopnea or flow limitation. For an AHI greater than or equal to 15 or 5, the patient may have the problems associated with daytime sleepiness, snoring, witnessed apnea and arousal due to temporary choking and gasping.

Generally, snoring refers to the symptom where annoying sound is caused during sleep by the vibration of the soft tissues in throat, velum or uvula when airflow passes through narrowed upper airway.

The respiration machines mentioned above has been recognized as the most effective way to treat abnormal respiration state, wherein patients may receive continuous gas supply from the respiration machines to increase blood oxygen concentration.

Respiration machines can be categorized into positive pressure respiration machines and negative pressure respiration machines according to the style of pressure supply.

For positive pressure respiration machines, a respiratory airflow of patients is detected as the basis for assisting patients to perform respiration activity at a proper time.

Implantable detection technique and non-implantable detection technique are currently employed to detect a respiratory airflow. A detector is implanted into human body, such as heart ventricle, lung, or larynx, as the implantable detection technique, and an external positive pressure respiration machine is used for detecting the airflow as the non-implantable detection technique. The non-implantable detection technique does not require implantation and therefore allows quicker and easier detection of respiratory airflow of the patient without causing much discomfort than the implantable counterpart.

Even though the non-implantable detection technique may enable simple detection, there are still some drawbacks in the conventional respiration machines because these machines fail to effectively and precisely identify the severity of the abnormal respiration state and may undesirably inhibit patient's respiration when continuously providing pressure to the patient, which may worsen the respiration disorder.

CN103961105A discloses a method and a system for performing snore recognition and strength output and a breathing machine. The system mainly comprises a breathing flow collector, a breathe recognizer, a snore feature recognizer and a snore strength output device which are connected in sequence, wherein the breathing flow collector collects expiration and inspiration flows in the breathing process and sends the flows to the breathe recognizer. By adopting the method and the system for performing the snore recognition and the strength output, the success rate of accurate judgment is improved, the hardware cost is saved, and the achieving complexity is reduced.

WO2013/138071A9 discloses a wearable breathing sensor, such as a piezoelectric chest belt system, generates a breathing signal that is analyzed by a classifier to identify apnea patterns indicating that the subject has swallowed during breathing. These breathing signals are computer-analyzed to extract inferences regarding the subject's eating and drinking patterns and thereby provide useful data for monitoring food or beverage intake for remote health monitoring.

Therefore, there is an unsolved need to provide a respiration machine allowing precise identification of severity and providing proper air pressure appropriately.

Accordingly, this disclosure provides a respiratory waveform recognition method and a system thereof capable of precisely identifying a normal respiration state and an abnormal respiration state from a respiratory air-

## SUMMARY OF THE INVENTION

A first objective of the present disclosure is to provide a respiratory waveform recognition method, in which an amplitude and a duration of a respiratory airflow are timely normalized to provide a normalized respiratory airflow useful for an algorithm to precisely calculate a flow index useful for the identification of a normal respiration state and a abnormal respiration state, wherein the abnormal respiration state includes apnea, hypopnea and flow limitation.

A second objective of the present disclosure is to provide a respiratory waveform recognition method which captures an inspiration waveform or an expiration waveform from the respiratory airflow to reduce the computation load and time of the algorithm, wherein the respiratory waveform recognition method may identify the state of the respiratory airflow by any one of the inspiration waveform and the expiration waveform.

A third objective of the present disclosure is to provide a respiratory waveform recognition method, in which a curve fitting method is used for fitting the normalized inspiration waveform or expiration waveform to a first-order linear curve, a high-order linear curve, a weighted curve or a standard waveform, so as to determine the severity of the abnormal respiration state.

A fourth objective of the present disclosure is to provide a respiratory waveform recognition method, in which several sampling points in the respiratory airflow are redistributed through weighting during the fitting process, so as to promote the identification of the severity of the abnormal respiration state.

A fifth objective of the present disclosure is to provide a respiratory waveform recognition method, in which one of a plurality of standard waveforms most similar to the normalized inspiration waveform or the normalized expiration waveform is selected to calculate the errors between the two waveforms to determine the severity of the abnormal respiration state.

A sixth objective of the present disclosure is to provide a respiratory waveform recognition method, in which a portion of the normalized inspiration waveform or the normalized expiration waveform is selected, and the differences, such as the standard deviation of an absolute error, between the portion of the waveform and one of the standard waveforms are calculated to determine the severity of the abnormal respiration state.

A seventh objective of the present disclosure is to provide a respiratory waveform recognition system for detecting a respiratory airflow and identifying a normal respiration state and an abnormal respiration state therefrom.

In order to achieve these and other objectives, the present invention provides a respiratory waveform recognition method useful for identification of a normal respiration state and an abnormal respiration state from a respiratory airflow, the respiratory waveform recognition method comprising: (a) detecting the respiratory airflow in a respiratory cycle; (b) measuring an amplitude of the respiratory airflow and a duration of the respiratory cycle; (c) using a plurality of sampling points to determine an inspiration waveform and an expiration waveform according to the amplitude and the duration; (d) normalizing the amplitude and the duration of one of the inspiration waveform and the expiration waveform, so as to establish a normalized waveform; and (e) accumulating the differences between the normalized waveform and a reference waveform to calculate a flow index useful for the identification of the normal respiration state and the abnormal respiration state, characterized in that the reference waveform is a second-order polynomial or a third-order polynomial.

Also disclosed is a respiratory waveform recognition method for identification of a normal respiration state and an abnormal respiration state from a respiratory airflow, the respiratory waveform recognition method comprising: (a1) detecting the respiratory airflow in a respiratory cycle; (b1) measuring an amplitude of the respiratory airflow and a duration of the respiratory cycle; (c1) using a plurality of sampling points to determine an inspiration waveform and an expiration waveform according to the amplitude and the duration; (d1) normalizing the amplitude and the duration of one of the inspiration waveform and the expiration waveform, so as to establish a normalized waveform; (e1) fitting the normalized waveform to a polynomial; and (f1) using a weighted least-squares method to calculate the differences between the normalized waveform and the polynomial, and calculating a flow index from the differences with a weighting function, the flow index being useful for the identification of the normal respiration state and the abnormal respiration state.

Also disclosed is a respiratory waveform recognition method for identification of a normal respiration state and an abnormal respiration state from a respiratory airflow, the respiratory waveform recognition method comprising: (a2) detecting the respiratory airflow in a respiratory cycle; (b2) measuring an amplitude of the respiratory airflow and a duration of the respiratory cycle; (c2) using a plurality of sampling points to determine an inspiration waveform and an expiration waveform according to the amplitude and the duration; (d2) normalizing the amplitude and the duration of one of the inspiration waveform and the expiration waveform, so as to establish a normalized waveform; (e2) comparing a plurality of standard waveforms and the normalized waveform, so as to set one of the standard waveforms as a reference waveform which is most similar to the normalized waveform among the standard waveforms; and (f2) accumulating the differences between the normalized waveform and a reference waveform to calculate a flow index useful for the identification of the normal respiration state and the abnormal respiration state.

In order to achieve these and other objectives, the present invention provides a respiratory waveform recognition system for identification of a normal respiration state and an abnormal respiration state from a respiratory airflow, the respiratory waveform recognition system comprising an airflow sensor, a processing unit and a display unit. The airflow sensor detects flow variation of the respiratory airflow for a duration. The processing unit is connected to the airflow sensor for receiving a signal corresponding to the flow variation of the respiratory airflow, using an algorithm to analyze an amplitude of the respiratory airflow and measuring a duration of a respiratory cycle to calculate a flow index, the algorithm being adapted to determine, according to the flow index, whether the respiratory airflow represents the normal respiration state or the abnormal respiration state. The display unit is connected to the processing unit for displaying the normal respiration state or the abnormal respiration state. The algorithm samples the amplitude and the duration of a plurality of sampling points, the amplitude and the duration determine an inspiration waveform and an expiration waveform, and the algorithm normalizes the amplitude and the duration of one of the inspiration waveform and the expiration waveform, so as to establish a normalized waveform. The algorithm is configured for obtaining the flow index by calculating the differences between a reference waveform and the normalized waveform, or by calculating the differences between a polynomial and the normalized waveform, characterized in that the reference waveform is a second-order polynomial or a third-order polynomial.

In brief, the respiratory waveform recognition method and system of the present disclosure may fit the waveform of a respiratory airflow with at least one of multiple curves as defined in the independent claims and calculate the differences therebetween. A flow index is calculated according to the differences, and the flow index may determine whether the respiratory airflow represents the normal respiration state or the abnormal respiration state. In addition to the determination of different respiration states, the flow index may further identify the severity of the abnormal respiration state, such as apnea, hypopnea, or flow limitation.

Notably, for hypopnea or flow limitation, the method and system of the present disclosure may further derive an AHI index from the flow index so as to determine obstructive sleep apnea or snoring symptom.

The present disclosure solves the problems of conventional respiration machines which fail to effectively determine the severity of the abnormal respiration state and undesirably inhibit patient's respiration when continuously providing pressure to the patient, which may worsen the respiration disorder.

## BRIEF DESCRIPTION OF THE DRAWINGS

Many aspects of the present embodiments can be better understood with reference to the following drawings. Moreover, in the drawings, like reference numerals designate corresponding parts throughout the several views.

FIG. 1 illustrates a flowchart of one embodiment of the respiratory waveform recognition method according to the present disclosure;
FIG. 2(a) illustrates a waveform representing a normal respiration state;
FIG. 2(b) illustrates a waveform representing an abnormal respiration state;
FIG. 3 illustrates a waveform representing the abnormal respiration state of FIG. 2(b) after amplitude normalization and duration or time normalization;
FIG. 4 illustrates several standard waveforms $S(i)$; and
FIG. 5 illustrates a block diagram of one embodiment of the respiratory waveform recognition system according to the present disclosure.

## DETAILED DESCRIPTION OF THE INVENTION

Embodiments of the present disclosure will now be described in detail below and with reference to the drawings.

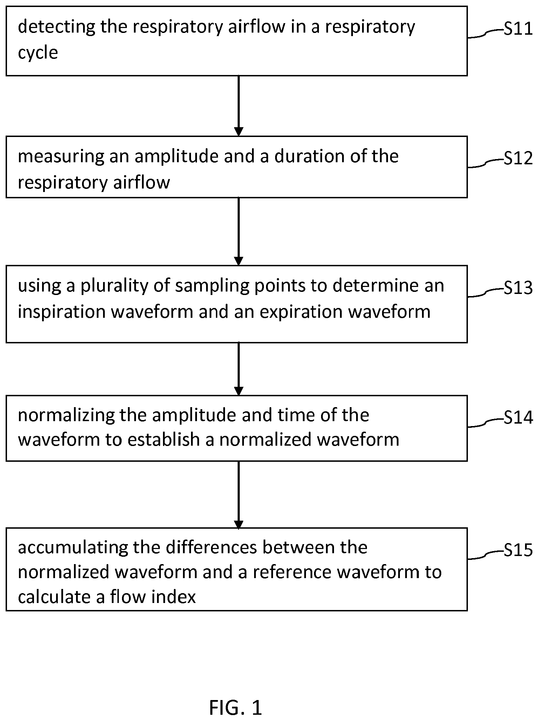

FIG. 1 illustrates a flowchart of one embodiment of the respiratory waveform recognition method according to the present disclosure, the respiratory waveform recognition method being capable of identifying and/or determining a normal respiration state and/or an abnormal respiration state from the waveform of a respiratory airflow.

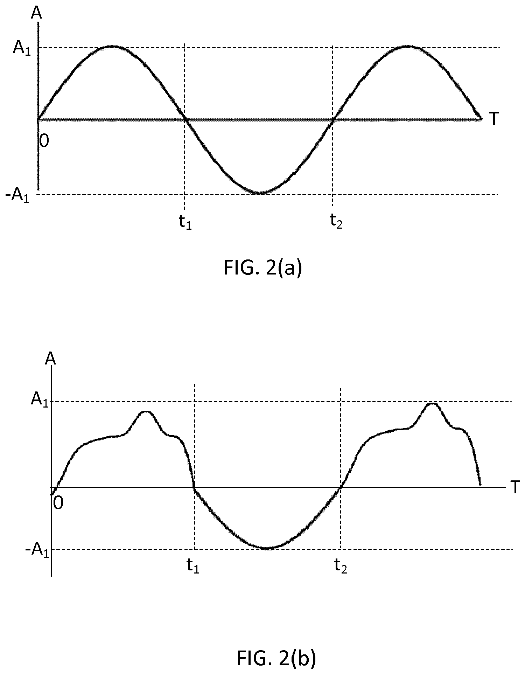

FIG. 2(a) illustrates a waveform representing a normal respiration state. FIG. 2(a) may also represent the waveform reflecting the variation of airflow in the airway of a patient during inspiration and expiration, wherein the x-axis represents time T and the y-axis represents amplitude A. The waveform between time 0 and $t_1$ may represent the inspiration airflow variation, and the waveform between time $t_1$ and $t_2$ may represent expiration airflow variation. In the normal respiration state, the inspiration airflow variation is substantially equal to the expiration airflow variation.

FIG. 2(b) illustrates a waveform representing an abnormal respiration state. In the abnormal respiration state, the airway is influenced by different obstruction degrees, so the inspiration airflow variation is not equal to the expiration airflow variation.

Referring back to FIG. 1, the respiratory waveform recognition method starts from step S11 for detecting the respiratory airflow in a respiratory cycle. Taking FIG. 2(b) as the example, the respiratory cycle is defined as the duration of completing one inspiration and one expiration, i.e. the cycle from time 0 to $t_2$.

Step S12 is adapted for measuring an amplitude of the respiratory airflow and a duration of the respiratory cycle. In FIG. 2(b), the amplitude represents the magnitude between $A_1$ to $-A_1$, and the duration represents the span between time 0 to $t_2$.

Step S13 is adapted for using a plurality of sampling points to determine an inspiration waveform (from time 0 to $t_1$) and an expiration waveform (from time $t_1$ to $t_2$) according to the amplitude A and the duration T. For example, sampling at a low frequency, such as several Hz, may be employed to create corresponding sampling points on the coordinate of amplitude A and corresponding time T, so as to delineate the waveform.

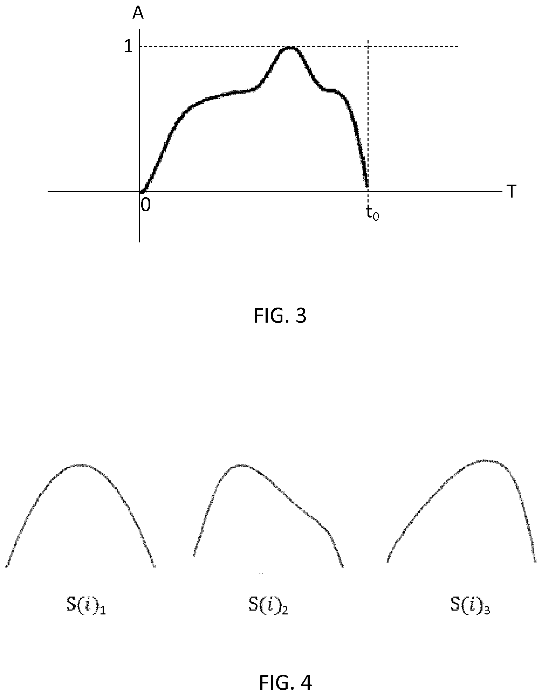

Step S14 is adapted for normalizing the amplitude A and the time T of one of the inspiration waveform and the expiration waveform, so as to establish a normalized waveform, as shown in FIG. 3, which represents a normalized inspiration waveform. In FIG. 3, because the amplitude of the inspiration waveform has been normalized, the magnitude of the amplitude A is normalized from $A_1$ to 1, and the length of time T is normalized from $t_1$ to to.

Step S15 is adapted for accumulating the differences, such as the least absolute error value and/or the least square error value, between the normalized waveform and a reference waveform to provide a flow index for determining whether the normalized waveform is identified as the normal respiration state or the abnormal respiration state. Several different types of reference waveforms may be used, as described below.

a) First-order polynomial (embodiment not covered by the claimed invention)

The reference waveform may be a first-order polynomial $\alpha_1 \cdot i + \alpha_2$, wherein the flow index has the mathematical representation $\sum_{i=x}^{y} |C1(i) - F(i)|$ wherein C1(i) is the first-order polynomial, $\alpha_1$ and $\alpha_2$ are constants, x is a starting point of the sampling points, y is an ending point of the sampling points, $i$ is each sampling point between x and y, and $F(i)$ is the amplitude A of the normalized waveform at the sampling point i.

b) Second-order polynomial

The reference waveform may be a second-order polynomial $\alpha_1 \cdot i^2 + \alpha_2 \cdot i + \alpha_3$, wherein the flow index has the mathematical representation $\sum_{i=x}^{y} |C2(i) - F(i)|$, wherein C2(i) is the second-order polynomial, $a_1$, $\alpha_2$ and $a_3$ are constants, x is a starting point of the sampling points, y is an ending point of the sampling points, $i$ is each sampling point between x and y, and $F(i)$ is the amplitude A of the normalized waveform at the sampling point i.

Because the second-order polynomial is plotted as a curve, it provides better fitting than the first-order polynomial.

c) Third-order polynomial

The reference waveform may be a third-order polynomial $\alpha_1 \cdot i^3 + \alpha_2 \cdot i^2 + a_3 \cdot i + a_4$, wherein the flow index has the mathematical representation $\sum_{i=x}^{y} |C3(i) - F(i)|$, wherein C3(i) is the third-order polynomial, $a_1$, $\alpha_2$, $a_3$ and $a_4$ are constants, x is a starting point of the sampling points, y is an ending point of the sampling points, $i$ is each sampling point between x and y, and $F(i)$ is the amplitude A of the normalized waveform at the sampling point $i$.

Compared with the fitting effect of the second-order polynomial, the third-order polynomial could further provide the capability of reflecting left or right shifting of the curve.

d) Third-order polynomial with weighting

The reference waveform may be a third-order polynomial $\alpha_1 \cdot i^3 + \alpha_2 \cdot i^2 + a_3 \cdot i + a_4$, wherein the flow index has the mathematical representation $\sum_{i=x}^{y} W(i) \times |C4(i) - F(i)|$, wherein $W(i)$ is defined below:

$$W(i) \begin{cases} A & , i = x, y \\ B & , i = MAX(F(i)); \\ C & , others \end{cases}$$

wherein C4(i) is the third-order polynomial, $a_1$, $\alpha_2$, $a_3$ and $a_4$ are constants, x is a starting point of the sampling points, y is an ending point of the sampling points, $i$ is each sampling point between x and y, $W(i)$ is a weighting function, A, B and C are weighting factors, and $F(i)$ is the amplitude A of the normalized waveform at the sampling point $i$.

Compared with the fitting effect of the third-order polynomial, the third-order polynomial with the weighting could further weight for example the starting point x or the ending point y so as to adjust the end points of the curve delineated by the third-order polynomial and reduce the error caused by the end points.

In this embodiment, the third-order polynomial with the weighting cites only the starting point and the ending point as the example, but this is for illustrative purpose only and not for limiting. In other words, other sampling points may also be weighted in addition to or instead of the starting point and the ending point.

In one embodiment, the weighting factor A ranges from 50 to 100, the weighting factor B ranges from 200 to 400, and the weighting factor C is 1.

e) Standard waveform

The reference waveform may also be one of a plurality of standard waveforms S($i$), as illustrated in FIG. 4, in which waveforms S($i$)$_1$, S($i$)$_2$ and S($i$)$_3$ of three normal respiration states are exemplified. The flow index has the following mathematical representation:

$$\sqrt{\frac{\sum_{i=x}^{y}(Ki-\mu)^2}{L}}\ ;$$

wherein

$$Ki = \sum_{i=x}^{y} R \times |F(i) - S(i)|\ ;$$

$$\mu = \frac{1}{L}\sum Ki\ ;$$

$$R = \frac{\sum S(i)}{\sum F(i)}\ ;$$

wherein x is a starting point of the sampling points, y is an ending point of the sampling points, $i$ is each sampling point between x and y, L is the duration between the starting point and the ending point, K$i$ is an absolute error between any one of the standard waveforms and the normalized waveform, $\mu$ is the mean of absolute errors, $R$ is a ratio of area under curve of any one of the standard waveforms and the normalized waveform, and $F(i)$ is the amplitude A of the normalized waveform at the sampling point $i$.

In one embodiment, the starting point is set to 20% of the sampling points and the ending point is set to 80% of the sampling points.

In various embodiments of the reference waveforms a) to e), a plurality of flow thresholds can be set for the flow index. By comparing the flow index with the flow thresholds, the airway obstruction degree can be further determined from the flow index, such as clear airway, mild obstruction, severe obstruction or snoring.

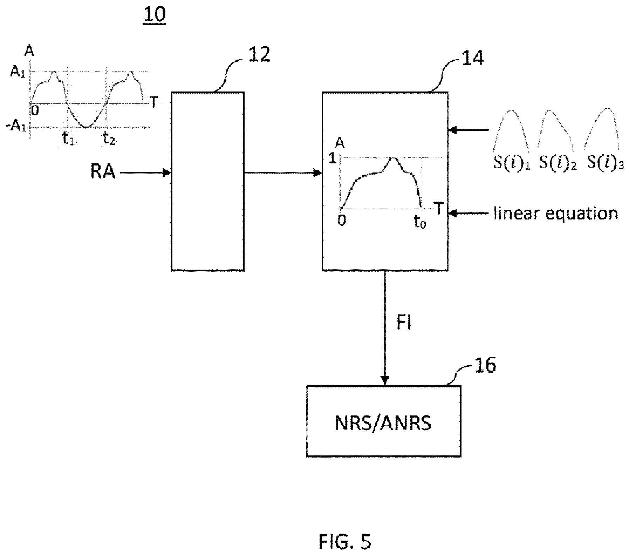

FIG. 5 illustrates a block diagram of one embodiment of the respiratory waveform recognition system according to the present disclosure. The respiratory waveform recognition system 10 is capable of identifying a normal respiration state NRS and an abnormal respiration state ANRS from a respiratory airflow RA.

The respiratory waveform recognition system 10 comprises an airflow sensor 12, a processing unit 14 and a display unit 16.

In a period of time, the airflow sensor 12 detects the flow variation of the respiratory airflow RA, and the length of the period or duration is sufficient for detecting the ascending waveform or descending waveform of the respiratory airflow RA.

The processing unit 14 is connected with the airflow sensor 12. The processing unit 14 receives a flow variation signal of the respiratory airflow RA and employs an algorithm to analyze the amplitude A of the respiratory airflow RA and measure the time T of the respiratory cycle, thereby obtaining a flow index FI. Moreover, the algorithm determines whether the respiratory airflow RA represents the normal respiration state NRS or the abnormal respiration state ANRS according to the flow index FI.

For example, the algorithm obtains amplitudes A and times T of a plurality of sampling points. The amplitudes A and times T determines an inspiration waveform and an expiration waveform. The algorithm normalizes the amplitudes A and times T of one of the inspiration waveform and the expiration waveform, so as to establish a normalized waveform. The algorithm calculates the differences between a standard waveform and the normalized waveform, or the differences between a polynomial and the normalized waveform, so as to obtain the flow index FI.

In another embodiment, the reference waveform is selected from a plurality of standard waveforms, and the selected standard waveform is most similar to the normalized waveform.

The display unit 16 is connected with the processing unit 14. The display unit 16 displays the normal respiration state NRS or the abnormal respiration state ANRS.

In another embodiment, the respiratory waveform recognition system 10 further comprises a storage unit (not shown) connected with the processing unit 12 to store the flow index FI.

**Claims**

1. A respiratory waveform recognition method useful for identification of a normal respiration state and an abnormal respiration state from a waveform of a respiratory airflow, the respiratory waveform recognition method comprising:

   detecting the respiratory airflow in a respiratory cycle;
   measuring an amplitude of the respiratory airflow and a duration of the respiratory cycle;
   using a plurality of sampling points to determine an inspiration waveform and an expiration waveform according to the amplitude and the duration;
   normalizing the amplitude and the duration of one of the inspiration waveform and the expiration waveform, so as to establish a normalized waveform; and
   accumulating the differences between the normalized waveform and a reference waveform to calculate a flow index useful for the identification of the normal respiration state and the abnormal respiration state, **characterized in that** the reference waveform is a second-order polynomial or a third-order polynomial.

2. The respiratory waveform recognition method of claim 1, wherein the second-order polynomial C2(i) has the following mathematical representation:

$$a_1 \cdot i^2 + a_2 \cdot i + a_3 \; ;$$

and the flow index has the following mathematical representation:

$$\sum\nolimits_{i=x}^{y} |C2(i) - F(i)| \; ;$$

wherein $\alpha_1$, $\alpha_2$ and $a_3$ are constants, x is a starting point of the sampling points, y is an ending point of the sampling points, $i$ is each sampling point between x and y, and $F(i)$ is the amplitude of the normalized waveform at the sampling point i.

3. The respiratory waveform recognition method of claim 1, wherein the third-order polynomial C3(i) has the following mathematical representation:

$$a_1 \cdot i^3 + a_2 \cdot i^2 + a_3 \cdot i + a_4 \; ;$$

and the flow index has the following mathematical representation:

$$\sum\nolimits_{i=x}^{y} |C3(i) - F(i)| \; ;$$

wherein $\alpha_1$, $\alpha_2$, $a_3$ and $a_4$ are constants, x is a starting point of the sampling points, y is an ending point of the sampling points, $i$ is each sampling point between x and y, and $F(i)$ is the amplitude of the normalized waveform at the sampling point i.

4. The respiratory waveform recognition method of claim 1, wherein the third-order polynomial C4(i) with a weighting has the following mathematical representation:

$$a_1 \cdot i^3 + a_2 \cdot i^2 + a_3 \cdot i + a_4 \; ;$$

and the flow index has the following mathematical representation:

$$\sum\nolimits_{i=x}^{y} W(i) \times |C4(i) - F(i)|;$$

wherein

$$W(i) \begin{cases} A & , i = x, y \\ B & , i = MAX(F(i)) \; ; \\ C & , others \end{cases}$$

wherein $\alpha_1$, $\alpha_2$, $a_3$ and $a_4$ are constants, x is a starting point of the sampling points, y is an ending point of the sampling points, $i$ is each sampling point between x and y, $W(i)$ is a weighting function, A, B and C individually represent a weighting factor, and $F(i)$ is the amplitude of the normalized waveform at the sampling point $i$.

5. The respiratory waveform recognition method of claim 4, wherein the weighting factor A ranges from 50 to 100, the weighting factor B ranges from 200 to 400, and the weighting factor C is 1.

6. The respiratory waveform recognition method of claim 1, wherein the step of accumulating the differences between the normalized waveform and the reference waveform comprises:
using a weighted least-squares method to calculate the differences.

7. The respiratory waveform recognition method of claim 1, wherein the differences represent at least one of a least absolute error value or a least square error value.

8. The respiratory waveform recognition method of claim 1, wherein the abnormal respiration state indicates apnea, hypopnea or flow limitation.

9. A respiratory waveform recognition system (10) for identification of a normal respiration state (NRS) and an abnormal respiration state (ANRS) from a respiratory airflow (RA), the respiratory waveform recognition system (10) comprising an airflow sensor (12), a processing unit (14) and a display unit (16);

the airflow sensor (12) detecting flow variation of the respiratory airflow (RA) for a duration;
the processing unit (14) being connected to the airflow sensor (12), the processing unit (14) receiving a signal corresponding to the flow variation of the respiratory airflow (RA), using an algorithm to analyze an amplitude (A) of the respiratory airflow (RA) and measuring the duration to calculate a flow index (FI), the algorithm being adapted to determine, according to the flow index (FI), whether the respiratory airflow (RA) represents the normal respiration state (NRS) or the abnormal respiration state (ANRS); and
the display unit (16) is connected to the processing unit (14), the display unit (16) displaying the normal respiration state (NRS) or the abnormal respiration state (ANRS);

wherein the algorithm samples the amplitude (A) and the duration of a plurality of sampling points, the amplitude (A) and the duration determine an inspiration waveform and an expiration waveform, and the algorithm normalizes the amplitude (A) and the duration of one of the inspiration waveform and the expiration waveform, so as to establish a normalized waveform, the algorithm obtaining the flow index (FI) by calculating the differences between a reference waveform and the normalized waveform, **characterized in that** the reference waveform is a second-order polynomial or third-order polynomial.

10. The respiratory waveform recognition system (10) of claim 9, further comprising a storage unit connected to the processing unit (14) for storing the flow index.

**Patentansprüche**

1. Ein Verfahren zur Erkennung von Atmungswellenformen, das bei der Identifizierung eines normalen Atmungszustands und eines abnormales Atmungszustands von einer Wellenform eines Atemluftstroms behilflich ist, wobei das Verfahren zur Erkennung von Atmungswellenformen Folgendes umfasst:

Erfassung des Atemluftstroms in einem Atemzyklus;
Messen einer Amplitude des Atemluftstrom und einer Dauer des Atemzyklus;
Verwendung einer Pluralität von Probenentnahmestellen, um eine Einatmungswellenform und eine Ausatmungswellenform nach der Amplitude und der Dauer zu bestimmen;
Normalisierung der Amplitude und der Dauer einer Einatmungswellenform und Ausatmungswellenform, um eine normalisierte Wellenform zu ermitteln; und
Akkumulierung der Unterschiede zwischen der normalisierten Wellenform und einer Bezugswellenform, um den Flussindex zu bestimmen, der für die Identifizierung des normalen Atmungszustands und des abnormalen Atmungszustands nützlich ist, **dadurch gekennzeichnet, dass** die Bezugswellenform ein Polynom zweiten Grades oder ein Polynom dritten Grades ist.

2. Das Verfahren zur Erkennung von Atmungswellenformen nach Anspruch 1, wobei das Polynom zweiten Grades C2 (i) die folgende mathematische Form aufweist:

$$a_1 \cdot i^2 + a_2 \cdot i + a_3 ;$$

und wobei der Flussindex die folgende mathematische Form aufweist:

$$\sum_{i=x}^{y} |C2(i) - F(i)| ;$$

wobei $a_1$, $a_2$ und $a_3$ Konstanten sind, wobei x der Startpunkt der Probenentnahmestellen und y der Endpunkt der Probenentnahmestellen ist, wobei $i$ jeder Probenentnahmepunkt zwischen x und y ist, und wobei $F(i)$ die Amplitude der normalisierten Wellenform am Probenentnahmepunkt $i$ ist.

3. Das Verfahren zur Erkennung von Atmungswellenformen nach Anspruch 1, wobei das Polynom dritten Grades C3(i) die folgende mathematische Form aufweist:

$$a_1 \cdot i^3 + a_2 \cdot i^2 + a_3 \cdot i + a_4 ;$$

und wobei der Flussindex die folgende mathematische Form aufweist:

$$\sum_{i=x}^{y} |C3(i) - F(i)| ;$$

wobei $a_1$, $a_2$, $a_3$ und $a_4$ Konstanten sind, wobei x der Startpunkt der Probenentnahmestellen und y der Endpunkt der Probenentnahmestellen ist, wobei $i$ jeder Probenentnahmepunkt zwischen x und y ist, und wobei $F(i)$ die Amplitude der normalisierten Wellenform am Probenentnahmepunkt $i$ ist.

4. Das Verfahren zur Erkennung von Atmungswellenformen nach Anspruch 1, wobei das Polynom dritten Grades C4(i) mit einer Gewichtung die folgende mathematische Form aufweist:

$$a_1 \cdot i^3 + a_2 \cdot i^2 + a_3 \cdot i + a_4 ;$$

und wobei der Flussindex die folgende mathematische Form aufweist:

$$\sum_{i=x}^{y} W(i) \times |C4(i) - F(i)| ;$$

wobei

$$W(i) \begin{cases} A & , i = x, y \\ B & , i = MAX(F(i)) ; \\ C & , others \end{cases}$$

wobei $a_1$, $\alpha_2$, $a_3$ und $a_4$ Konstanten sind, wobei x der Startpunkt der Probenentnahmestellen und y der Endpunkt der Probenentnahmestellen ist, wobei $i$ jeder Probenentnahmepunkt zwischen x und y ist, wobei W(i) eine Gewichtungsfunktion ist, und wobei A, B und C einzeln einen Gewichtungsfaktor darstellen und wobei $F(i)$ die Amplitude der normalisierten Wellenform am Probenentnahmepunkt $i$ ist.

5. Das Verfahren zur Erkennung von Atmungswellenformen nach Anspruch 4, wobei der Gewichtungsfaktor A zwischen 50 und 100 und der Gewichtungsfaktor zwischen 200 und 400 liegt, wobei der Gewichtungsfaktor der Wert 1 aufweist.

6. Das Verfahren zur Erkennung von Atmungswellenformen nach Anspruch 1, wobei der Schritt der Akkumulierung der Unterschiede zwischen der normalisierten Wellenform und der Bezugswellenform Folgendes umfasst:
Verwendung eines gewichteten Verfahrens der kleinsten Quadrate zur Berechnung der Unterschiede.

7. Das Verfahren zur Erkennung von Atmungswellenformen nach Anspruch 1, wobei die Unterschiede mindesten einen kleinsten absoluten Fehlerwert oder einen kleinsten quadratischen Fehlerwert darstellen.

8. Das Verfahren zur Erkennung von Atmungswellenformen nach Anspruch 1, wobei der abnormale Atmungszustand eine Apnoe, eine Hypopnoe oder eine Atemflussbegrenzung anzeigt.

9. Ein System zur Erkennung von Atmungswellenformen (10) für die Identifizierung eines normalen Atmungszustands (NRS) und eines abnormalen Atmungszustands (ANRS) vom Atemluftstrom (RA), wobei das System zur Erkennung der Atmungswellenform (10) einen Luftflusssensor (12), eine Verarbeitungseinheit (14) und eine Anzeigeeinheit (16) umfasst;

Wobei der Luftflusssensor (12) die Variation des Atemluftflusses (RA) für eine Dauer feststellt;
Wobei die Verarbeitungseinheit (14) mit dem Luftflusssensor (12) verbunden ist und die Verarbeitungseinheit (14) ein Signal empfängt, das der Luftflussvariation des Atemluftflusses (RA) entspricht und einen Algorithmus verwendet, um eine Amplitude (A) des Atemluftflusses (RA) zu analysieren und eine Dauer zu messen, um einen Flussindex (FI) zu berechnen, wobei der Algorithmus angepasst wird, um entsprechend dem Flussindex (FI) zu bestimmen, ob der Atemluftfluss (RA) einen normalen Atmungszustand (NRS) oder einen abnormalen Atmungszustand (ANRS) darstellt; und
Wobei die Anzeigeeinheit (16) mit der Verarbeitungseinheit (14) verbunden ist du die Anzeigeeinheit (16) den normalen Atmungszustand (NRS) oder den abnormalen Atmungszustand (ANRS) anzeigt;
wobei der Algorithmus die Amplitude (A) und die Dauer einer Vielzahl der Probenentnahmestellen abfragt, wobei die Amplitude (A) und die Dauer eine Einatmungswellenform und eine Ausatmungswellenform bestimmen und der Algorithmus die Amplitude (A) und die Dauer einer der Einatmungswellenformen und der Ausatmungswellenformen normalisieren, um eine normalisierte Wellenform zu erhalten, wobei der Algorithmus den Flussindex (FI) bestimmt, indem er die Unterschiede zwischen der Bezugswellenform und der normalisierten Wellenform errechnet, **dadurch gekennzeichnet, dass** die Bezugswellenform ein Polynom zweiten Grades oder ein Polynom dritten Grades ist.

10. Das System zur Erkennung von Atmungswellenformen (10) nach Anspruch 9, das ferner eine Speichereinheit umfasst, die mit der Verarbeitungseinheit (14) verbunden ist, um den Flussindex zu speichern.

## Revendications

1. Procédé de reconnaissance de la forme d'onde respiratoire utile pour l'identification d'un état respiratoire normal et d'un état respiratoire anormal à partir d'une forme d'onde d'un flux d'air respiratoire, le procédé de reconnaissance de la forme d'onde respiratoire comprenant :

la détection du flux d'air respiratoire dans un cycle respiratoire ;
la mesure d'une amplitude du flux d'air respiratoire et d'une durée du cycle respiratoire ;
l'utilisation d'une pluralité de points d'échantillonnage pour déterminer une forme d'onde d'inspiration et une forme d'onde d'expiration en fonction de l'amplitude et de la durée ;
la normalisation de l'amplitude et de la durée de l'une des formes d'onde d'inspiration et de la forme d'onde d'expiration, de manière à établir une forme d'onde normalisée ; et
l'accumulation des différences entre la forme d'onde normalisée et une forme d'onde de référence pour calculer un indice d'écoulement utile à l'identification de l'état respiratoire normal et de l'état respiratoire anormal, **caractérisé en ce que** la forme d'onde de référence est un polynôme de deuxième ordre ou un polynôme de troisième ordre.

**2.** Procédé de reconnaissance de forme d'onde respiratoire selon la revendication 1, dans lequel le polynôme de deuxième ordre C2(i) a la représentation mathématique suivante :

$$a_1 \cdot i^2 + a_2 \cdot i + a_3 \; ;$$

et 1"indice de débit a la représentation mathématique suivante :

$$\sum_{i=x}^{y} |C2(i) - F(i)| \; ;$$

dans lequel ai, $\alpha_2$ et $a_3$ sont des constantes, x est un point de départ des points d'échantillonnage, y est un point d'arrêt des points d'échantillonnage, i est chaque point d'échantillonnage entre x et y, et F(i) est l'amplitude de la forme d'onde normalisée au point d'échantillonnage i.

**3.** Procédé de reconnaissance de forme d'onde respiratoire selon la revendication 1, dans lequel le polynôme de troisième ordre C3(i) a la représentation mathématique suivante :

$$a_1 \cdot i^3 + a_2 \cdot i^2 + a_3 \cdot i + a_4 \; ;$$

et 1"indice de débit a la représentation mathématique suivante :

$$\sum_{i=x}^{y} |C3(i) - F(i)| \; ;$$

dans lequel ai, $\alpha_2$, $a_3$ et $a_4$ sont des constantes, x est un point de départ des points d'échantillonnage, y est un point d'arrêt des points d'échantillonnage, i est chaque point d'échantillonnage entre x et y, et F(i) est l'amplitude de la forme d'onde normalisée au point d'échantillonnage i.

**4.** Procédé de reconnaissance de forme d'onde respiratoire selon la revendication 1, dans lequel le polynôme de troisième ordre C4(i) a la représentation mathématique suivante :

$$a_1 \cdot i^3 + a_2 \cdot i^2 + a_3 \cdot i + a_4 \; ;$$

et 1"indice de débit a la représentation mathématique suivante :

$$\sum_{i=x}^{y} W(i) \times |C4(i) - F(i)| \; ;$$

où

$$W(i) \begin{cases} A & , i = x, y \\ B & , i = MAX(F(i)) \; ; \\ C & , autres \end{cases}$$

dans lequel ai, $\alpha_2$, $a_3$ et $a_4$ sont des constantes, x est un point de départ des points d'échantillonnage, y est un point d'arrêt des points d'échantillonnage, i est chaque point d'échantillonnage entre x et y, W(i) est une fonction de pondération, A, B et C représentent individuellement un facteur de pondération, et F(i) est l'amplitude de la forme d'onde normalisée au point d'échantillonnage i.

**5.** Procédé de reconnaissance de la forme d'onde respiratoire selon la revendication 4, dans lequel le facteur de pondération A varie de 50 à 100, le facteur de pondération B de 200 à 400, et le facteur de pondération C est 1.

**6.** Procédé de reconnaissance de forme d'onde respiratoire selon la revendication 1, dans lequel l'étape d'accumulation des différences entre la forme d'onde normalisée et la forme d'onde de référence comprend :
l'utilisation d'une méthode pondérée des moindres carrés pour calculer les différences.

**7.** Procédé de reconnaissance de la forme d'onde respiratoire selon la revendication 1, dans lequel les différences représentent au moins une valeur d'erreur absolue ou une valeur d'erreur de moindre carré.

**8.** Procédé de reconnaissance de la forme d'onde respiratoire selon la revendication 1, dans lequel l'état respiratoire anormal indique une apnée, une hypopnée ou une limitation du débit.

**9.** Système de reconnaissance de la forme d'onde respiratoire (10) pour l'identification d'un état respiratoire normal (NRS) et d'un état respiratoire anormal (ANRS) à partir d'un flux d'air respiratoire (RA), le système de reconnaissance de la forme d'onde respiratoire (10) comprenant un capteur de débit d'air (12), une unité de traitement (14) et une unité d'affichage (16) ;

le capteur de débit d'air (12) détectant la variation du débit d'air respiratoire (RA) pendant une durée ;
l'unité de traitement (14) étant connectée au capteur de débit d'air (12), l'unité de traitement (14) recevant un signal correspondant à la variation de débit du flux d'air respiratoire (RA), utilisant un algorithme pour analyser une amplitude (A) du flux d'air respiratoire (RA) et mesurant la durée pour calculer un indice de débit (FI), l'algorithme étant adapté pour déterminer, selon l'indice de débit (FI), si le flux d'air respiratoire (RA) représente l'état respiratoire normal (NRS) ou l'état respiratoire anormal (ANRS) ; et l'unité d'affichage (16) est connectée à l'unité de traitement (14), à l'unité d'affichage (16) affichant l'état de respiration normal (NRS) ou l'état de respiration anormal (ANRS) ;

dans lequel l'algorithme échantillonne l'amplitude (A) et la durée d'une pluralité de points d'échantillonnage, l'amplitude (A) et la durée déterminent une forme d'onde d'inspiration et une forme d'onde d'expiration, et l'algorithme normalise l'amplitude (A) et la durée de l'une des formes d'onde d'inspiration et de la forme d'onde d'expiration, de manière à établir une forme d'onde normalisée, l'algorithme obtenant l'indice d'écoulement (FI) en calculant les différences entre une forme d'onde de référence et la forme d'onde normalisée, **caractérisé en ce que** la forme d'onde de référence est un polynôme de deuxième ordre ou un polynôme de troisième ordre.

10. Système de reconnaissance de forme d'onde respiratoire (10) selon la revendication 9, comprenant en outre une unité de stockage reliée à l'unité de traitement (14) pour stocker l'indice de débit.

| detecting the respiratory airflow in a respiratory cycle | S11 |
|---|---|

| measuring an amplitude and a duration of the respiratory airflow | S12 |

| using a plurality of sampling points to determine an inspiration waveform and an expiration waveform | S13 |

| normalizing the amplitude and time of the waveform to establish a normalized waveform | S14 |

| accumulating the differences between the normalized waveform and a reference waveform to calculate a flow index | S15 |

FIG. 1

FIG. 2(a)

FIG. 2(b)

FIG. 3

$S(i)_1$          $S(i)_2$          $S(i)_3$

FIG. 4

FIG. 5

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103961105 A
- WO 2013138071 A9